# EUROPÄISCHE PATENTSCHRIFT

(11) **EP 4 452 117 B1**
(45) Veröffentlichungstag und Bekanntmachung des Hinweises auf die Patenterteilung: **02.09.2026**
(21) Anmeldenummer: 22838674.4
(22) Anmeldetag: 13.12.2022
(51) Int. Cl.: A61B 34/20, A61B 90/30, A61B 34/30

(54) **ROBOTERGESTÜTZTES REGISTRIERUNGSVERFAHREN UND CHIRURGISCHES NAVIGATIONSSYSTEM**
ROBOT-SUPPORTED REGISTRATION METHOD AND SURGICAL NAVIGATION SYSTEM
PROCÉDÉ D'ENREGISTREMENT ASSISTÉ PAR ROBOT ET SYSTÈME DE NAVIGATION CHIRURGICALE

(30) Priorität: 23.12.2021 DE 102021134553
(43) Veröffentlichungstag der Anmeldung: 30.10.2024
(73) Patentinhaber: B. Braun New Ventures GmbH, 79110 Freiburg im Breisgau (DE)
(72) Erfinder: DIPPEL, Maximilian, 79111 Freiburg (DE); STAWIASKI, Jean, 79199 Kirchzarten (DE)
(74) Vertreter: Winter, Brandl - Partnerschaft mbB
(86) Internationale Anmeldenummer: PCT/EP2022/085625
(87) Internationale Veröffentlichungsnummer: WO 2023/117587

(56) Entgegenhaltungen:
- WO-A1-2021/146339
- US-A1- 2018 055 578
- US-A1- 2019 107 700

## Beschreibung

### Technisches Gebiet

Die vorliegende Offenbarung betrifft ein Registrierungsverfahren/ Registrierverfahren zur Registrierung eines Patienten und/oder einer Aufnahme eines Patienten gegenüber einem Navigationssystem, insbesondere gegenüber 3D-Aufnahmedaten. Daneben betrifft die Offenbarung ein chirurgisches Navigationssystem für einen chirurgischen Eingriff bei einem Patienten zur Registrierung und zur Nachverfolgung sowie ein computerlesbares Speichermedium gemäß den Oberbegriffen der nebengeordneten Ansprüche.

### Hintergrund der Offenbarung

Üblicherweise werden bei einer chirurgischen Navigation besondere Instrumente verwendet, welche spezielle Markierungssysteme mit Markern, wie etwa Infrarot-Referenzpunkte, aufweisen. Diese Instrumente werden als Zeiger oder Pointer bezeichnet und sind speziell dafür angepasst, mit ihrer Spitze eine Position im dreidimensionalen Raum zu markieren, welche durch ein Navigationssystem erfasst wird. Insbesondere werden solche navigierten Pointer zum Abtasten von Landmarken im Gesicht eines Patienten verwendet, um eine Registrierung des Patienten und/oder einer Aufnahme des Patienten gegenüber dem zu verwendenden Navigationssystem durchzuführen.

Eine weitere Alternative einer Registrierung besteht in dem Einsatz einer nachverfolgten (Video)Kamera in Kombination mit einer Navigationskamera/ Trackingkamera.

Diese vorstehend genannten Registrierungsverfahren haben jedoch den Nachteil, dass sie in einer Bauchlage des Patienten nur sehr schlecht funktionieren, da hierbei das Gesicht des Patienten nach unten zeigt. In diesem Fall müssen die Chirurgen in die Knie gehen und sich unter den Patienten begeben, um das Gesicht des Patienten für eine Registrierung zu erfassen, was jedoch zu Problemen mit einem Sichtbereich oder einer (optischen) Sichtlinie zwischen dem eingesetzten und manuell geführten Pointer oder Videokamera und der Trackingkamera/ Nachverfolgungskamera/ Navigationskamera führt. Um dennoch einen Sichtkontakt zwischen der Trackingkamera und dem nachverfolgten Pointer oder der nachverfolgten Kamera herzustellen, müssen die Chirurgen die Navigationskamera/ Trackingkamera während der Registrierung oft mehrmals neu positionieren, so dass ein Sichtkontakt wiederhergestellt wird. Dies verzögert einen Ablauf eines Eingriffs und birgt zudem Sicherheitsrisiken, sowohl für den Patienten als auch für das medizinische Fachpersonal.

Alternativ zu der Registrierung in Bauchlage kann der Patient in einer Rückenlage oder einer Seitenlage registriert werden und dann für den Eingriff in die Bauchlage gedreht werden. Diese Methode ist jedoch sehr riskant und erhöht die Komplexität des chirurgischen Arbeitsablaufs außerordentlich.

Zudem ist die manuelle Registrierung eine Frage des Könnens des Einzelnen. Auf Basis unterschiedlicher individueller Erfahrungen und Kompetenzen kann daher eine Qualität der Registrierung variieren, je nachdem, welche Person die Registrierung durchführt. Die manuelle Registrierung ist sehr zeitaufwändig, insbesondere dann, wenn die Registrierung aufgrund mangelnder Erfahrung mehrmals wiederholt werden muss, um ein hinreichendes Ergebnis zu erhalten.
US 2019/107700 A1 offenbart ein System zur Steuerung eines robotergeführten Operationsmikroskops um eine Bildstabilität zu verbessern, wobei eine Registrierung mittels einer Oberfläche des Gesichts durchgeführt ist.
US 2018/055578 A1 offenbart ein medizinisches Navigationssystem, das eine Computervorrichtung mit einem Prozessor, eine Verfolgungskamera zum Verfolgen medizinischer Geräte und eine Anzeige zum Anzeigen eines Bildes; eine automatisierte Armanordnung, die elektrisch mit der Computervorrichtung gekoppelt ist und durch ein von der Computervorrichtung bereitgestelltes Signal gesteuert wird, wobei die automatisierte Armanordnung einen Mehrgelenkarm mit einem distalen Ende umfasst, das mit einem Effektor verbunden werden kann, der eine mit der Computervorrichtung elektrisch gekoppelte chirurgische Kamera trägt.

### Zusammenfassung der Offenbarung

Es sind daher die Aufgaben der vorliegenden Offenbarung, die Nachteile aus dem Stand der Technik zu vermeiden oder zumindest zu mindern und insbesondere ein Registrierungsverfahren, ein chirurgisches Navigationssystem und ein computerlesbares Speichermedium zur Verfügung zu stellen, das eine höhere Präzision einer Registrierung und einer Navigation liefert und insbesondere eine kontinuierliche und unterbrechungsfreie Nachverfolgung sowie vorzugsweise eine noch bessere Erfassung eines Eingriffsbereichs erlaubt. Ein Nutzer, insbesondere Chirurg, soll eine noch bessere und sicherere Registrierung und damit Navigationsmodalität erhalten. Zudem ist eine Teilaufgabe, einen chirurgischen Arbeitsablauf noch besser zu unterstützen und schneller durchzuführen. Eine weitere Teilaufgabe kann darin gesehen werden, eine zur Verfügung stehende Navigationszeit während eines Eingriffs zu erhöhen.

Die Aufgaben der vorliegenden Offenbarung werden hinsichtlich eines gattungsgemäßen Registrierungsverfahrens erfindungsgemäß durch die Merkmale des Anspruchs 1 gelöst, hinsichtlich eines gattungsgemäßen chirurgischen Navigationssystems erfindungsgemäß durch die Merkmale des Anspruchs 7 gelöst und hinsichtlich eines computerlesbaren Speichermediums erfindungsgemäß durch die Merkmale des Anspruchs 10 gelöst.

Ein Grundgedanke der vorliegenden Offenbarung kann also darin gesehen werden, dass für eine präzise Registrierung eines Patienten (und/oder einer Aufnahme eines Patienten) eine an einem Roboterarm vorgesehene optische Roboter-Kamera verwendet wird, die insbesondere ein Gesicht des Patienten räumlich erfasst und abtastet/ scannt. Diese optische, robotergeführte Roboter-Kamera wird wiederum von einem Trackingsystem, insbesondere mit und über eine Navigationskamera, in seiner Lage erfasst und nachverfolgt. Das Trackingsystem erfasst einerseits vorzugsweise den Patienten, etwa über einen Patienten-Tracker, und erfasst die Roboter-Kamera, etwa über einen Roboter-Kamera-Tracker. Die Roboter-Kamera kann durch den Roboter geführt werden, um relevante Körperabschnitte des Patienten, insbesondere des Gesichts, in verschiedenen Lagen aus verschiedenen Blickrichtungen zu scannen und entsprechende Aufnahmen zu erstellen. Sollte die Roboter-Kamera in einen RaumBereich geraten, in welcher kein direkter Sichtkontakt zwischen dem Trackingsystem mit der Trackingkamera und dem Roboterkopf mit der Roboter-Kamera, insbesondere mit dem Roboter-Tracker, besteht, so kann ein Roboterkinematikbasiertes Trackingsystem (Roboterkinematik-Trackingsystem) für eine Nachverfolgung der Lage der Roboter-Kamera hinzugezogen und ergänzt werden, um auch ohne direkten Sichtkontakt eine Lage des Roboterkopfs bzw. der Roboter-Kamera relativ zu dem Patienten und/oder relativ zu dem Navigationssystem, insbesondere zu der Trackingkamera, zu bestimmen. Da der Roboterkopf eine feste Transformation zu der Roboter-Kamera aufweist, kann von dem einen lokalen Koordinatensystem (KOS) auf das andere lokale KOS geschlossen werden bzw. eine Lage des Roboterkopfs lässt einen Rückschluss auf die Lage der Roboter-Kamera zu. Insbesondere ist eine Transformation von einem Roboter-Kamera-Tracker zu der Roboter-Kamera dem Navigationssystem bekannt oder lässt sich bestimmen.

Ein solches Registrierungsverfahren oder Navigationsverfahren und Navigationssystem bieten die Vorteile einer hohen Effizienz einer Registrierung als auch einer deutlichen Verbesserung einer Reproduzierbarkeit bzw. Wiederholung einer solchen Registrierung, da diese automatisch und robotergeführt erfolgen. Dadurch verringert sich insbesondere ein Stressfaktor für einen Chirurgen und Assistenten und ein Eingriff kann noch effizienter und schneller durchgeführt werden. Auch kann der Roboter des Navigationssystems während des Registriervorgangs vorzugsweise implizit auch eine Zugänglichkeit eines Arbeitsvolumens bzw. Eingriffsbereichs überprüfen. Insbesondere kann mit dem Registrierverfahren und dem Navigationssystem also als zusätzlicher Vorteil eine Zugänglichkeit eines chirurgischen Arbeitsbereichs für den Roboter bereits während der Registrierung überprüft werden. Auf diese Weise kann eine Neupositionierung des Roboters, etwa eines mobilen Roboters mit einem fahrbaren Wagen als Roboterbasis, während des Eingriffs vermieden werden.

Mit andere Worten wird insbesondere eine Registrierung im Bereich der Robotik und eine Navigation in der Chirurgie, insbesondere Neurochirurgie offenbart. Hierbei wird eine automatische und präzise Registrierung unter Verwendung einer an einem Roboterarm angebundenen oder montierten (Sicht-)Kamera (Roboter-Kamera) durchgeführt, die für eine Registrierung des Patienten zu dem Navigationssystem insbesondere das Gesicht des Patienten scannt. Diese (Sicht-)Kamera /Roboter-Kamera wird nachverfolgt, und zwar durch eine Kombination einer Verwendung einer Trackingkamera/ Navigationskamera des Navigationssystems und einem (integrierten) Roboternachverfolgungssystem (Roboterkinematik-Trackingsystem). Das chirurgische Navigationssystem der vorliegenden Offenbarung hat damit zwei unterschiedliche Nachverfolgungsmodalitäten zur Nachverfolgung der Roboter-Kamera zur Verfügung und kann die jeweils beste Modalität wählen oder auch eine Kombination für eine noch präzisere Registrierung verwenden.

Ein großes Problem, dass Assistenten und Chirurgen über unterschiedliche Fähigkeiten zur Durchführung einer Registrierung verfügen, entfällt bei dem vorliegenden Registrierungsverfahren und bei dem chirurgischen Navigationssystem gemäß der vorliegenden Offenbarung. Ebenso wenig müssen Registrierungen mehrfach durchgeführt oder zu einem späteren Zeitpunkt wiederholt werden, wenn etwa der Operateur mit der Arbeit seines Assistenten nicht zufrieden war, sondern insbesondere können, beispielsweise in regelmäßigen Zeitintervallen oder zu bestimmten Zeitpunkten während des Eingriffs (jederzeit) eine erneute automatische Registrierung durchgeführt werden, um sowohl eine Qualität einer Registrierung als auch eine Reproduzierbarkeit und Effizienz zu verbessern. Insbesondere kann zu allen möglichen Zeitpunkten während des Eingriffs eine automatische Registrierung durchgeführt werden, um die Qualität, Reproduzierbarkeit und Effizienz zu verbessern.

Während also beim Stand der Technik eine manuelle Registrierung mit einem händisch geführten Pointer oder einer nachverfolgten Videokamera eine mehrfache Neupositionierung einer Tracking-Kamera des Navigationssystems erfordert, wird bei dem vorliegenden Registrierungsverfahren und Navigationssystems ein Einsatz eines Roboters mit Roboterarm und einer an den Roboterarm angebundenen Roboter-Kamera zur Überwindung von Sichtlinienproblemen vorgesehen, ohne dass das externe Trackingsystem, insbesondere die Navigationskamera/ Trackingkamera, bewegt werden muss. Es wird also ein Einsatz eines Roboters bzw. Roboterarms zur automatischen Registrierung durch Scannen des Patienten und automatischen Durchführung des Abgleichs vorgesehen. Im Falle von Sichtlinienproblemen bzw. einer Unterbrechung einer Sichtlinie oder Sichtbereichs zwischen dem Trackingsystem und der nachverfolgten Roboter-Kamera wird das Roboterpositionierungssystem/ Roboter-Trackingsystem/ Roboterkinematik-Trackingsystem als Zwischenkoordinatenreferenz verwendet, um über das Roboterkinematik-Trackingsystem die Lage der Roboter-Kamera zu bestimmen. Auch wird ein Eingriff mit Navigationsverfahren noch sicherer, da eine Netto-Navigationszeit, welche dem Chirurgen zur Verfügung steht, weiter erhöht wird.

Mit anderen Worten wird ein Registrierungsverfahren für eine (automatische) Registrierung eines Patienten für eine chirurgische Navigation bei einem chirurgischen Eingriff offenbart, welches die Schritte aufweist: Vorzugsweise Erfassen einer Lage des Patienten, insbesondere eines Kopfs des Patienten, durch eine Trackingkamera, insbesondere eine 3D-Trackingkamera, eines Navigationssystems; Erfassen (einer Lage) eines Roboterkopfs mit einer Roboter-Kamera (eines medizinischen Roboters), insbesondere über einen Roboter-Kamera-Tracker, durch die Trackingkamera des Navigationssystems und Nachverfolgen der (Position und/oder Orientierung, insbesondere der Lage der) Roboter-Kamera; Steuern und Bewegen des Roboterarms derart, dass die Roboter-Kamera in einer ersten Lage auf einen interessierenden Bereich des Patienten, insbesondere auf ein Gesicht des Patienten, gerichtet ist und Erstellen einer Aufnahme durch die Roboter-Kamera; Prüfen, ob in der ersten Lage eine Sichtverbindung zwischen der Trackingkamera und dem Roboterkopf mit der Roboter-Kamera, insbesondere mit dem Roboter-Tracker, vorliegt; Erfassen, bei Vorliegen einer Sichtverbindung, einer Lage des Roboterkopfs mit der Roboterkamera über die Trackingkamera, oder Erfassen, bei Fehlen oder Unterbrechung oder Verminderung einer Sichtverbindung, einer Lage des Roboterkopfs mit der Roboterkamera über ein Roboterkinematik-Trackingsystems; und Durchführen einer Registrierung des Patienten und/oder der (durch die Roboter-Kamera erstellte) Aufnahme gegenüber dem Navigationssystem, insbesondere gegenüber der Trackingkamera. Insbesondere wird bei der Registrierung die Aufnahme, die durch die Roboter-Kamera erstellt wurde, in Koordinaten oder in ein Koordinatensystem der Trackingkamera überführt.

Man könnte auch sagen, dass das Registrierungsverfahren bzw. das Navigationssystem für eine Nachverfolgung (einer Lage) primär die Trackingkamera verwendet, jedoch bei einer Verschlechterung eines Sichtkontakts in einen zweiten Modus einer Nachverfolgung wechselt/ umswitched, nämlich in die Nachverfolgung auf Basis des Roboterkinematik-Trackingsystems.

Der Begriff "Position" meint eine geometrische Position im dreidimensionalen Raum, der insbesondere mittels Koordinaten eines kartesischen Koordinatensystems angegeben wird. Insbesondere kann die Position durch die drei Koordinaten X, Y und Z angegeben werden.

Der Begriff "Orientierung" wiederum gibt eine Ausrichtung (etwa an der Position) im Raum an. Man kann auch sagen, dass durch die Orientierung eine Ausrichtung angegeben wird mit Richtungs- bzw. Drehungsangabe im dreidimensionalen Raum. Insbesondere kann die Orientierung mittels drei Winkeln angegeben werden.

Der Begriff "Lage" umfasst sowohl eine Position als auch eine Orientierung. Insbesondere kann die Lage mittels sechs Koordinaten angegeben werden, drei Positionskoordinaten X, Y und Z sowie drei Winkelkoordinaten für die Orientierung.

Der Begriff 3D definiert dabei, dass die Daten oder Strukturen räumlich, also dreidimensional vorliegen. Der Körper des Patienten oder zumindest ein Teilbereich des Körpers mit räumlicher Ausdehnung kann in einem dreidimensionalen Raum mit etwa einem kartesischen Koordinatensystem (X, Y, Z) digital als Aufnahmedaten vorliegen.

Es wird insbesondere keine Trackingkamera des Navigationssystems benötigt, wenn nur das Roboterkinematik-Trackingsystem des Roboters verwendet wird. Insbesondere ist hierbei bereits eine Transformation zwischen der Roboterbasis und dem Patienten bekannt oder kann über die Roboter-Kamera bestimmt werden.

Vorteilhafte Ausführungsformen sind in den Unteransprüchen beansprucht und werden insbesondere nachfolgend erläutert.

Gemäß einer weiteren Variante kann das Registrierungsverfahren ferner die Schritte aufweisen: Steuern und Bewegen des Roboterarms derart, dass die Roboter-Kamera in zumindest einer zweiten Lage auf einen weiteren interessierenden Bereich des Patienten, insbesondere auf einen weiteren Gesichtsbereich des Patienten, gerichtet ist und Erstellen einer zweiten Aufnahme durch die Roboter-Kamera; Prüfen, ob in der zweiten Lage eine Sichtverbindung zwischen der Trackingkamera und dem Roboterkopf mit der Roboter-Kamera, insbesondere mit dem Roboter-Tracker, vorliegt; Erfassen, bei Vorliegen einer Sichtverbindung, der zweiten Lage des Roboterkopfs mit der Roboterkamera über die Trackingkamera, oder Erfassen, bei Fehlen oder Unterbrechung oder Verminderung einer Sichtverbindung, einer Lage des Roboterkopfs mit der Roboter-Kamera über das Roboterkinematik-Trackingsystems. Insbesondere kann das Registrierungsverfahren mehrere Lagen der Roboter-Kamera nacheinander durchlaufen und in jeder Lage eine entsprechende Aufnahme erstellen. Insbesondere kann eine kontinuierliche Bewegung mit kontinuierlichen nachverfolgten Lagen der Roboter-Kamera und kontinuierliche Aufnahmen, ähnlich einer Videoaufnahme, erstellt werden.

Vorzugsweise kann bei dem Schritt Erfassen der Lage, bei einem Fehlen oder einer Unterbrechung oder einer Verminderung der Sichtverbindung, die Erfassung der Lage des Roboterkopfs mit der Roboter-Kamera über das Roboterkinematik-Trackingsystem erfolgen, wobei eine Transformation zwischen einer Roboterbasis des Roboters und der Trackingkamera verwendet wird, um über die Transformation von der Trackingkamera zu der Roboterbasis und der Transformation des Roboterkinematik-Trackingsystems von der Roboterbasis zum Roboterkopf mit der Roboter-Kamera, eine Transformation zwischen dem lokalen Koordinatensystem der Trackingkamera und dem Koordinatensystem der Roboter-Kamera und damit deren Lage zu bestimmen.

Gemäß einer Ausführungsform kann das Registrierungsverfahren ferner den Schritt aufweisen: Erfassen einer relativen Lage des Roboterkopfs mit der Roboter-Kamera zu der Lage des Kopfs des Patienten über die Trackingkamera, um dem Roboter eine Orientierung für die automatische Registrierung bereitzustellen.

Insbesondere kann das Registrierungsverfahren ferner den Schritt aufweisen: Verwenden einer 3D-Kamera als Roboter-Kamera und Scannen eines Gesichts des Patienten, insbesondere eines Kopfs des Patienten, von mehreren Seiten, um eine Gesamtaufnahme des Gesichts, insbesondere des Kopfs zu erhalten.

Insbesondere kann das Registrierungsverfahren den Schritt aufweisen, Erfassen einer Lage der Roboterbasis und damit eines Referenzkoordinatensystems des Roboters und des Roboterkinematik-Trackingsystems durch die Trackingkamera. Insbesondere kann das Navigationssystem über die Trackingkamera ferner eine Lage des Patienten, insbesondere des Kopfs des Patienten, erfassen bzw. das Registrierungsverfahren diesen Schritt aufweisen, so dass eine Transformation zwischen dem lokalen Koordinatensystem (KOS) des Roboters und dem lokalen Koordinatensystem (KOS) des Patienten bekannt ist, und das Roboterkinematik-Trackingsystem kann hiernach unabhängig von der Trackingkamera agieren.

Gemäß einer weiteren Ausführungsform kann der Schritt der Registrierung des Patienten erst dann durchgeführt werden, wenn die Aufnahmen erstellt wurden und die Roboter-Kamera sich wieder im Sichtbereich der Trackingkamera befindet.

Hinsichtlich eines chirurgisches Navigationssystems für einen chirurgischen Eingriff bei einem Patienten, werden die Aufgaben der vorliegenden Offenbarung insbesondere dadurch gelöst, dass dieses aufweist: einen Roboter mit einer Roboterbasis als lokalen Anbindungspunkt, einem an die Roboterbasis angebundenen Roboterarm sowie einem an den Roboterarm angebundenen, insbesondere gelagerten, Roboterkopf mit einer Roboter-Kamera, so dass eine Lage (also eine Position und Orientierung) der Roboter-Kamera gegenüber der Roboterbasis einstellbar ist; ein Trackingsystem mit einer Trackingkamera, die für eine Nachverfolgung (einer Lage) von vorzugsweise eines Patienten, insbesondere eines Kopfs des Patienten, vorzugsweise über einen Patienten-Tracker, und für eine Nachverfolgung der Roboter-Kamera (10) angepasst ist, insbesondere über einen Roboter-Kamera-Tracker; ein Roboterkinematik-Trackingsystem, das dafür angepasst ist, eine relative Lage des Roboterkopfs mit der Roboter-Kamera gegenüber seiner Roboterbasis zu bestimmen; und einer Steuereinheit, die dafür angepasst ist, den Roboter zu steuern und eine Registrierung des Patienten durchzuführen, wobei die Steuereinheit konfiguriert ist: In einer ersten Lage der Roboter-Kamera eine Aufnahme durch die Roboter-Kamera zu erstellen, insbesondere von einem Gesicht des Patienten; bei Vorliegen einer Sichtverbindung zwischen der Trackingkamera und dem Roboterkopf mit der Roboter-Kamera, insbesondere einem Roboter-Kamera-Tracker, die Lage der Roboter-Kamera durch die Trackingkamera (insbesondere relativ zu dieser) zu erfassen oder, bei Fehlen oder Unterbrechung oder Verminderung der Sichtverbindung, die Lage der der Roboter-Kamera (im Raum) über das Roboterkinematik-Trackingsystem zu erfassen; und eine Registrierung des Patienten und/oder der Aufnahme der Roboter-Kamera gegenüber dem Navigationssystem, insbesondere gegenüber der Trackingkamera, durchzuführen. Insbesondere muss bei der Registrierung die durch die Roboter-Kamera erstellte Aufnahme in die Koordinaten bzw. das Koordinatensystem (KOS) des Navigationssystems, insbesondere der Trackingkamera, überführt werden. Insbesondere wird eine Transformation zwischen dem Koordinatensystem der Aufnahme der Roboter-Kamera und dem Koordinatensystem des Navigationssystems, insbesondere dem KOS der Trackingkamera, ermittelt.

Gemäß einer weiteren Ausführungsform kann das chirurgische Navigationssystem ein robotergeführtes Operationsmikroskop als Roboter aufweisen und ein Mikroskopkopf des Operationsmikroskops kann als Roboterkopf mit Roboter-Kamera fungieren. Mit anderen Worten kann insbesondere die für die Registrierung verwendete Roboter-Kamera ein Operationsmikroskop bzw. ein Mikroskopkopf des Operationsmikroskops mit einem optischen System und einem Sensor für eine entsprechende Aufnahme sein.

Vorzugsweise kann das chirurgische Navigationssystem ein infrarotbasiertes (optisches) Trackingsystem aufweisen und/oder ein elektromagnetisches (EM) bzw. EM-basiertes Trackingsystem aufweisen und/oder ein auf Machine-Vision-basiertes Trackingsystem aufweisen bzw. auf maschinellem Sehen basieren.

Hinsichtlich eines computerlesbares Speichermedium werden die Aufgaben dadurch erfüllt, indem dieses Befehle umfasst, die bei einer Ausführung durch einen Computer diesen veranlassen, die Verfahrensschritte des Registrierungsverfahrens gemäß der vorliegenden Ausführungsform auszuführen.

Jegliche Offenbarung im Zusammenhang mit dem Navigationssystem der vorliegenden Offenbarung gilt ebenso für das Registrierungsverfahren der vorliegenden Offenbarung wie auch umgekehrt.

### Kurzbeschreibung der Figuren

Die vorliegende Erfindung wird nachfolgend anhand bevorzugter Ausführungsbeispiele unter Bezugnahme auf die begleitenden Figuren näher erläutert. Es zeigen:
- Fig. 1: eine schematische Ansicht eines chirurgischen Navigationssystems gemäß einer bevorzugten Ausführungsform, welche ein Infrarot-Tracking zur Erfassung einer Transformation zwischen einem Roboterkopf und einem Patientenkopf verwendet;
- Fig. 2: eine weitere perspektivische Ansicht des Navigationssystems aus Fig. 1, wobei die Kamera mit Tiefenwahrnehmung/ Abstandserfassung verwendet wird, um den Patienten von allen relevanten Seiten her zu scannen;
- Fig. 3: eine weitere perspektivische Ansicht des Navigationssystems aus Fign. 1 und 2, wobei bei Sichtlinienproblemen mit dem Roboter-Tracker vorübergehend die Roboterbasis als Referenz verwendet wird und über die Kinematik die Lage des Roboterkopfs bestimmt wird;
- Fig. 4: eine weitere perspektivische Ansicht des Navigationssystems aus Fign. 1 bis 3, wobei eine Registrierung durchgeführt wird, sobald der Scan abgeschlossen und der Roboter-Tracker wieder sichtbar ist; und
- Fig. 5: ein Flussdiagramm eines Registrierungsverfahrens einer bevorzugten Ausführungsform.

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

Fign .1 bis 4 zeigen, zur Erläuterung der vorliegenden Offenbarung und dessen Prinzipien, in einer schematischen perspektivischen Ansicht ein chirurgisches Navigationssystem 1 zur Navigation bei einem chirurgischen Eingriff bei einem Patienten P gemäß einer bevorzugten Ausführungsform, welche eine automatische Registrierung eines Patienten durchführen kann.

Das Navigationssystem 1 weist dafür einen Roboter 2 mit einer Roboterbasis 4 als lokalen Anbindungspunkt auf. An die Roboterbasis 4 ist ein mehrgelenkiger, Roboterarm 6 mit mehreren Roboterarm-Segmenten angebunden, um eine Artikulation zu ermöglichen. An den Roboterarm 6 wiederum ein endständiger Roboterkopf 8, der im Bereich des Patienten an unterschiedlichen Positionen in unterschiedlichen Orientierungen verfahrbar ist. Der Roboterkopf 8 weist wiederum eine stirnseitige Roboter-Kamera 10 auf, welche optische Aufnahmen, hier dreidimensionale Aufnahmen, also 3D-Aufnahmen mit Tiefeninformationen, erstellen kann. Über den Roboterarm 6 und den Roboterkopf 8 lässt sich auch entsprechend eine Lage, also eine Position und Orientierung der Roboter-Kamera 10 gegenüber der Roboterbasis 4 und damit auch gegenüber dem Patienten P einstellen.

Für eine Erfassung einer Position und einer Lage von Objekten, weist das Navigationssystem 1 ein infrarotbasiertes Trackingsystem 12 auf, das eine Trackingkamera 14 an einer solchen Stelle in einem Operationssaal hat, dass es einen guten Überblick über den Eingriffsbereich hat, insbesondere erhöht angeordnet ist. Die Trackingkamera 14 ist vorliegend eine 3D-Trackingkamera in Form einer Stereo-Kamera, um über zwei zueinander beabstandeten Kameras und eine entsprechende Verarbeitung der Aufnahmen, insbesondere über eine Triangulation, eine Lage eines nachzuverfolgenden Objekts erfasst werden kann.

In dieser Ausführungsform sind an den nachzuverfolgenden Objekten Infrarot (IR) Marker/ IR-Marker bzw. IR-Tracker angeordnet. Diese IR-Marker weisen vier starr zueinander beabstandet angeordnete IR-Kugeln für eine Erfassung der Position und Orientierung auf. Konkret ist an einem Kopf des Patienten ein Patienten-Tracker 16 in Form eines IR-Trackers angeordnet und ferner ist an dem Roboterkopf 8 mit der Roboter-Kamera 10 ein Roboter-Kamera-Tracker 18 angeordnet. Damit kann die (Stereo-)Trackingkamera 14 des Navigationssystems 1 über eine vordefinierte Relation von Tracker zum Kopf des Patienten bzw. zur Roboter-Kamera die Lage des Patienten und die Lage des Roboter-Kamera 10 bestimmen und nachverfolgen.

Als weiteren Unterschied zum Stand der Technik weist das vorliegende Navigationssystem 1 neben dem optischen, infrarotbasierten Trackingsystem 12 auch noch ein Roboterkinematik-Trackingsystem 20 des Roboters auf, das dafür angepasst ist, eine relative Lage des Roboterkopfs 8 mit der Roboter-Kamera 10 gegenüber seiner Roboterbasis 4 zu bestimmen. Konkret sind in dem Roboter 2 an den Gelenken Sensoren verbaut, welche eine Gelenkstellung des Roboters 2 erfassen und diese Einstellungen der Gelenke computerlesbar bereitstellen. Eine Verarbeitungseinheit, hier durch eine zentrale Steuereinheit 22 ausgebildet, bestimmt dann auf Basis der Erfassten Stellung des Roboters die Lage des Roboterkopfs 8 und über eine bekannte Transformation von Roboterkopf 8 zu Roboter-Kamera 10 dann die relative Lage (des lokalen Koordinatensystems (KOS)) der Roboter-Kamera 10 gegenüber (dem lokalen Koordinatensystem (KOS)) der Roboterbasis 4.

Die Steuereinheit 22 ist ferner dafür angepasst den Roboter 2 zu steuern und eine Registrierung des Patienten P durchzuführen, wobei die Steuereinheit 22 konfiguriert ist, die Roboter-Kamera 10 in eine erste Lage zu steuern und zu bewegen und in dieser ersten Lage der Roboter-Kamera 10 eine Aufnahme durch die Roboter-Kamera 10 zu erstellen, und zwar von einem Gesicht des Patienten P. Es wird also eine 3D-Oberflächenaunahme durch die Roboter-Kamera 10 in einer ersten Lage erstellt.

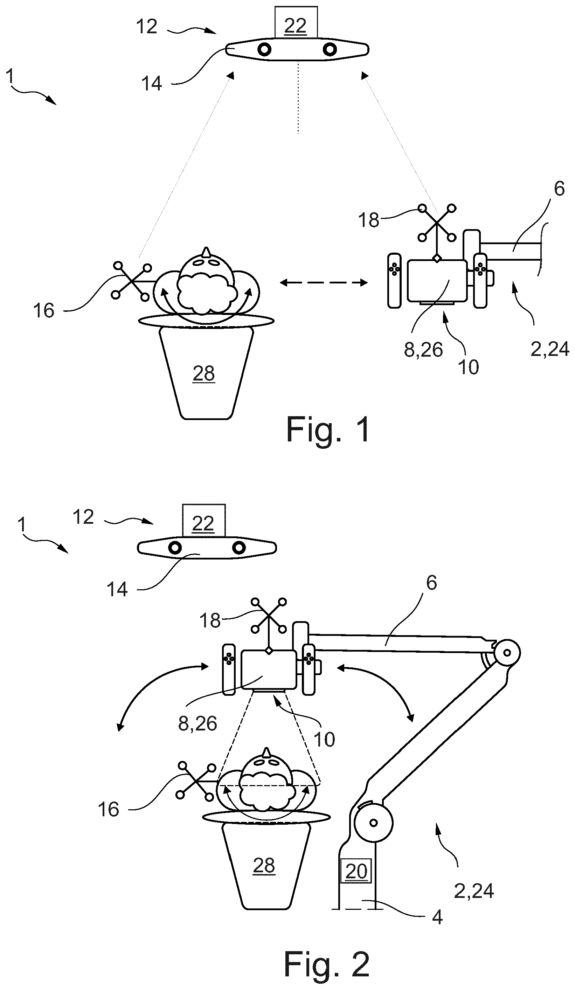

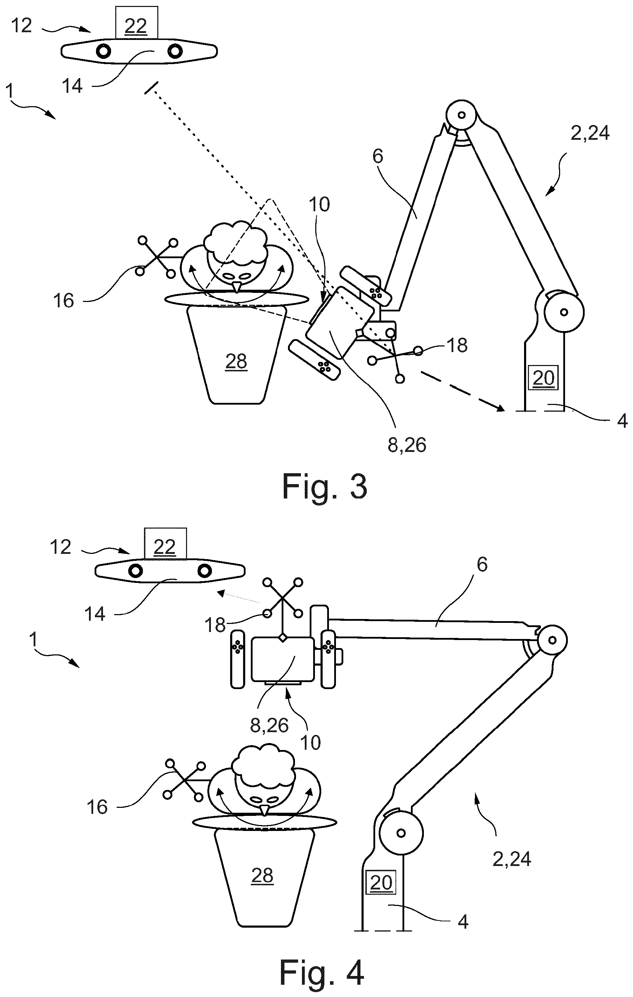

Wenn nun, wie in Fign. 2 und 3 gezeigt, eine direkte Sicht/ Sichtverbindung /Sichtlinie zwischen der Trackingkamera 14 und dem Roboter-Kamera-Tracker 18 besteht, also wenn Trackingsystem 12 über die Trackingkamera 14 den Roboter-Kamera-Tracker 18 und damit den Roboterkopf 8 mit der Roboter-Kamera 10 hinreichend gut orten kann, so verwendet das Navigationssystem für die Erfassung der Lage der Roboter-Kamera 10 im Raum das Trackingsystem 12 mit der Trackingkamera 14. Hierdurch wird einerseits die relative Lage der Roboter-Kamera 10 gegenüber dem lokalen Koordinatensystem (KOS) des Trackingsystem 2 bzw. der Trackingkamera 14 ermittelt, als auch, da auch die Lage des Patienten P bzw. zumindest eines Körperabschnitts des Patienten P, hier der Kopf des Patienten P, gegenüber dem Trackingsystem 12 bzw. der Trackingkamera 14 erfasst wurde, kann auch die Lage der Roboter-Kamera 10 gegenüber dem Patienten P bestimmt werden. Insbesondere kann dann nämlich auch eine Lage einer 3D-Oberflächenaufnahme der Roboter-Kamera 10 gegenüber dem Patienten bestimmt werde und für eine Registrierung verwendet werden. Damit wird bei Vorliegen einer Sichtverbindung zwischen der Trackingkamera 14 und dem Roboterkopf 8 mit der Roboter-Kamera 10, hier dem Roboter-Kamera-Tracker 18, die Lage der Roboter-Kamera 10 gegenüber dem Navigationssystem 1, insbesondere dem Trackingsystem, durch die Trackingkamera 14 erfasst.

Fehlt hingegen eine solche Sichtverbindung oder wird diese unterbrochen oder so stark beeinträchtigt / vermindert, so weist das vorliegende Navigationssystem 1 neben dem optischen Trackingsystem noch ein Roboterbasiertes bzw. Kinematikbasiertes Trackingsystem, nämlich das Roboterkinematik-Trackingsystem 20 auf. Dieses stellt eine relative Lage der Roboter-Kamera 10 gegenüber der Roboterbasis 4 bereit. Das Trackingsystem 12 kann über eine vorbestimmte Transformation zwischen Trackingkamera 14 und Roboterbasis 4 oder über die direkte Erfassung der Roboterbasis 4 in Sichtverbindung/ Sichtlinie, insbesondere über einen Roboter-Basis-Tracker (hier nicht dargestellt), eine Transformation zwischen dem lokalen Koordinatensystem des Trackingsystems 12 bzw. der Trackingkamera 14 und dem lokalen KOS der Roboterbasis 4 bestimmen. Damit kann in solchen Lagen des Roboterkopfs 8 und der Roboterkamera 10, in welchen die Sichtlinie zwischen der Trackingkamera 14 und der Roboter-Kamera 10 bzw. indirekt über den Roboter-Kamera-Tracker 18 gestört ist (siehe Fig. 3), auf das Roboterkinematik-Trackingsystem 20 zurückgreifen und eine Registrierung des Patienten gegenüber dem Navigationssystem 1 automatisch durchführen (siehe Fig. 4).

In der Ausführungsform gemäß Fign. 1 bis 4 weist das Navigationssystem 1 ein robotergeführtes Operationsmikroskop 24 als Roboter 2 auf. Ein Mikroskopkopf 26 des Operationsmikroskops 24 mit dem optischen System und dem Aufnahmesensor (hier nicht dargestellt, da in dem Mikroskopkopf 26 innenliegend) dient als Roboterkopf 8 mit Roboterkamera 10.

In Fig. 1 wird durch das Navigationssystem zunächst das infrarotbasierte Tracking zur Bewertung einer räumlichen, geometrischen Beziehung und Transformation zwischen dem Roboterkopf 8 und dem Kopf des Patienten P verwendet, um dem Roboter den möglichen Arbeitsbereich aufzuzeigen und den Roboter 2 entsprechend zu positionieren.

In Fig. 2 wird dann die Roboter-Kamera 10 (3D-Aufnahmekamera bzw. Kamera mit Tiefenwahrnehmung/ Tiefenerfassung) dafür eingesetzt, den Patienten, insbesondere den Kopf des Patienten P, bevorzugt das Gesicht des Patienten P, von allen relevanten Seiten her zu scannen und eine 3D-Oberflächenaufnahme von zumindest dem Gesicht zu erstellen. Da in den Positionen oberhalb des Patienten P der Roboter-Kamera-Tracker 18 stets in direktem Sichtkontakt zu der Trackingkamera 14 steht, kann diese auch die Lage der Roboter-Kamera 10 gegenüber dem Trackingsystem 12 bzw. der Trackingkamera 14 (etwa eine Linse der Trackingkamera 14 als lokales KOS des Trackingsystems) bestimmen.

Wird nun, wie in Fig. 3 dargestellt, der Patient vor oder während eines Eingriffs in die Bauchlage gelegt bzw. gedreht, so entsteht eine Sichtlinienproblematik, da die Roboter-Kamera 10, wenn sie das nach unten gerichtete Gesicht des Patienten P erfasst, unterhalb des Patienten P angeordnet ist und der Patient P oder ein Operationstisch 28 oder ein anderes medizinisches Personal oder gar der Roboter 2 selbst eine Sichtverbindung zu der Trackingkamera 14 blockiert. Bei einer solchen Sichtlinienproblemen mit dem Roboter-Kamera-Tracker 18 wird daher vorübergehend die Roboterbasis 4 als Referenz verwendet und über das Roboterkinematik-Trackingsystem 20 die Lage der Roboter-Kamera 10 gegenüber dem Patienten P erfasst. In der vorliegenden Ausführungsform wird die Lage der Roboter-Kamera 10 über die Roboterbasis und weiter über die Trackingkamera 14 hin zu dem Patienten P erfasst.

Sind nun alle relevanten Aufnahmen des Gesichts des Patienten P erstellt und der 3d-Oberflächen-scan abgeschlossen, so wird, sobald der Roboter-Kamera-Tracker 18 wieder in dem Sichtfeld der Trackingkamera 14 ist, die Registrierung durchgeführt und abgeschlossen.

Dieser Vorgang der Registrierung kann zu verschiedenen Zeitpunkten vor oder während des Eingriffs wiederholt werden, um eine besonders genaue Registrierung zu erreichen.

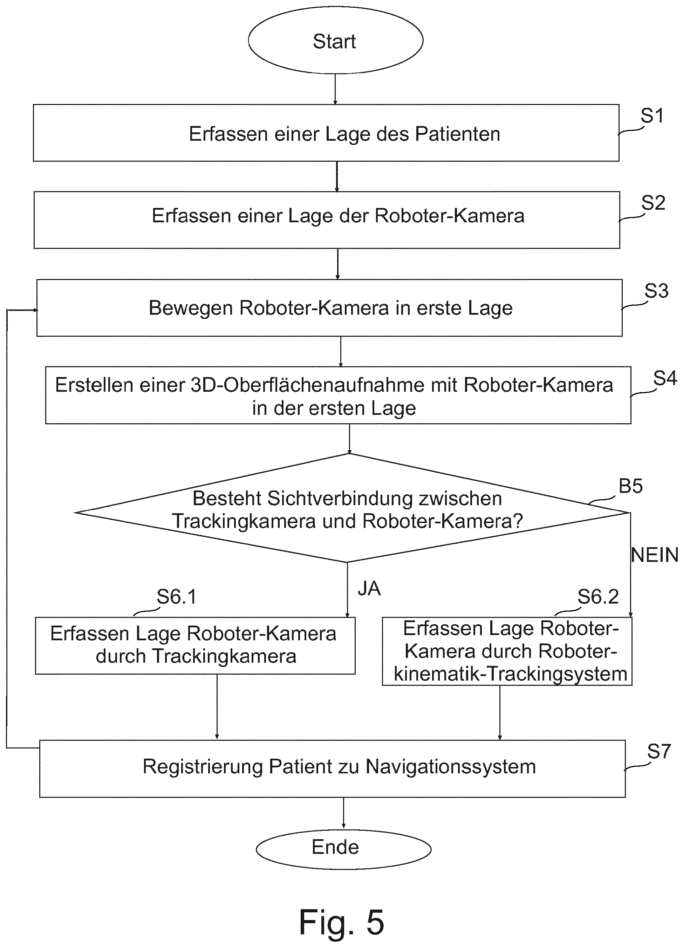

Fig. 5 zeigt in einem Flussdiagramm ein Registrierungsverfahren gemäß einer bevorzugten Ausführungsform, welches bei einem chirurgischen Navigationssystem, insbesondere bei einem vorstehend beschriebenen Navigationssystem 1, durchgeführt werden kann.

Das Registrierungsverfahren für eine automatische Registrierung eines Patienten P für eine chirurgische Navigation eines chirurgischen Eingriffs hat als einen (hier ersten) Schritt S1 ein Erfassen einer Lage des Patienten P, insbesondere eines Kopfs des Patienten P, durch eine Trackingkamera 14, insbesondere eine 3D-Trackingkamera, eines chirurgischen Navigationssystems 1.

In einem nachfolgenden Schritt S2 erfolgt ein Erfassen einer Lage eines Roboterkopfs 8 mit einer Roboter-Kamera 10 eines medizinischen Roboters 2, über einen Roboter-Kamera-Tracker 18, durch die Trackingkamera 14 des Navigationssystems 1 und Nachverfolgen der Roboter-Kamera 10.

In einem Schritt S3 wir der Roboterarm derart angesteuert und entsprechend bewegt, dass die Roboter-Kamera 10 in einer ersten Lage auf einen interessierenden Bereich des Patienten P, insbesondere auf ein Gesicht des Patienten P, gerichtet ist.

In einem Schritt S4 wird dann einer Aufnahme, hier einer 3D-Oberflächenaufnahme, durch die 3D-fähige Roboter-Kamera 10 erstellt.

In einer Bedingung B5 wird die Bedingung geprüft, ob in der ersten Lage eine Sichtverbindung zwischen der Trackingkamera 14 und dem Roboterkopf 8 mit der Roboter-Kamera 10, hier eine Sichtverbindung zu dem Roboter-Kamera-Tracker 18, vorliegt.

Wenn die Prüfung positiv ist, und eine Sichtverbindung vorliegt, so schreitet das Registrierungsverfahren zu Schritt 6.1 voran, welches die Lage des Roboterkopfs 8 mit der Roboter-Kamera 10 über die Trackingkamera 14 erfasst, hier über den Roboter-Kamera-Tracker 18.

Ist die Sichtverbindung unterbrochen oder stark beeinträchtigt, dass nicht mehr mit hinreichender Genauigkeit die Lage über die Trackingkamera 14 bestimmbar ist, so wird in einem Schritt S6.2 die Lage des Roboterkopfs 8 mit der Roboterkamera 10 über ein Roboterkinematik-Trackingsystem 20 bestimmt. Hierbei kann entweder ganz ohne Trackingkamera 14 eine Lage der Roboter-Kamera 10 durch das Roboterkinematik-Trackingsystem 20 zu dem Patienten P bestimmt werden, oder es kann implizit über Einbindung der Trackingkamera 14 und eine entsprechende Berechnung von Roboter-Kamera 10 - Roboterkopf 8 - Roboterbasis 4 -Trackingkamera 14 - Patient P die Transformation bereitgestellt werden.

Schließlich erfolgt in einem Schritt S7, wenn die Roboter-Kamera 10 wieder im Sichtfeld der Trackingkamera 14 ist, eine Registrierung des Patienten P gegenüber dem Navigationssystem 1.

### Bezugszeichenliste

- 1: Chirurgisches Navigationssystem
- 2: Roboter
- 4: Roboterbasis
- 6: Roboterarm
- 8: Roboterkopf
- 10: Roboter-Kamera
- 12: Trackingsystem
- 14: Trackingkamera
- 16: Patienten-Tracker
- 18: Roboter-Kamera-Tracker
- 20: Roboterkinematik-Trackingsystem
- 22: Steuereinheit
- 24: Operationsmikroskop
- 26: Mikroskopkopf
- 28: Operationstisch

- P: Patient

- S1: Schritt Erfassen einer Lage des Patienten
- S2: Schritt Erfassen einer Lage der Roboter-Kamera
- S3: Schritt Bewegen Roboter-Kamera in erste Lage
- S4: Schritt Erstellen einer Aufnahme
- B5: Bedingung Besteht Sichtverbindung?
- S6.1: Schritt Erfassen Lage Roboter-Kamera durch Trackingkamera
- S6.2: Schritt Erfassen Lage Roboter-Kamera durch Roboterkinematik-Trackingsystem
- S7: Schritt Registrierung Patient

## Patentansprüche

1. Registrierungsverfahren für eine automatische Registrierung eines Patienten (P) für eine chirurgische Navigation, aufweisend die Schritte:
Vorzugsweise Erfassen einer Position und/oder Orientierung (S1) des Patienten (P), insbesondere eines Kopfs des Patienten (P), durch eine Trackingkamera (14), insbesondere eine 3D-Trackingkamera, eines chirurgischen Navigationssystems (1);
Erfassen einer Lage (S2) eines Roboterkopfs (8) mit einer Roboter-Kamera (10) eines medizinischen Roboters (2), insbesondere über einen Roboter-Kamera-Tracker (18), durch die Trackingkamera (14) des Navigationssystems (1) und Nachverfolgen der Roboter-Kamera (10);
Steuern und Bewegen (S3), durch eine Steuereinheit (22), eines Roboterarms (6) derart, dass die Roboter-Kamera (10) in einer ersten Lage auf einen interessierenden Bereich des Patienten (P), insbesondere auf ein Gesicht des Patienten (P), gerichtet ist und Erstellen (S4) einer Aufnahme, insbesondere einer 3D-Aufnahme, durch die Roboter-Kamera (10);
Prüfen (B5), durch die Steuereinheit (22), ob in der ersten Lage eine Sichtverbindung zwischen der Trackingkamera (14) und dem Roboterkopf (8) mit der Roboter-Kamera (10), insbesondere mit dem Roboter-Kamera-Tracker (18), vorliegt;
Erfassen (S6.1), bei Vorliegen einer Sichtverbindung, einer Lage des Roboterkopfs (8) mit der Roboter-Kamera (10) über die Trackingkamera (14), oder Erfassen (S6.2), bei Fehlen oder Unterbrechung oder Verminderung einer Sichtverbindung, einer Lage des Roboterkopfs (8) mit der Roboterkamera (10) über ein Roboterkinematik-Trackingsystem (20); und
Durchführen (S7) einer Registrierung des Patienten und/oder der Aufnahme gegenüber dem Navigationssystem (1), insbesondere gegenüber der Trackingkamera (14).

2. Registrierungsverfahren nach Anspruch 1, **dadurch gekennzeichnet, dass** das Registrierungsverfahren ferner die Schritte aufweist:
Steuern und Bewegen des Roboterarms (6) derart, dass die Roboter-Kamera (10) in einer zweiten Lage auf einen weiteren interessierenden Bereich des Patienten (P), insbesondere auf einen weiteren Gesichtsbereich des Patienten (P), gerichtet ist und Erstellen einer zweiten Aufnahme durch die Roboter-Kamera (10);
Prüfen, ob in der zweiten Lage eine Sichtverbindung zwischen der Trackingkamera (14) und dem Roboterkopf (8) mit der Roboter-Kamera (10), insbesondere mit dem Roboter-Kamera-Tracker (18), vorliegt;
Erfassen, bei Vorliegen einer Sichtverbindung, der zweiten Lage des Roboterkopfs (8) mit der Roboterkamera (10) über die Trackingkamera (14), oder Erfassen, bei Fehlen oder Unterbrechung oder Verminderung einer Sichtverbindung, einer Lage des Roboterkopfs (8) mit der Roboter-Kamera (10) über das Roboterkinematik-Trackingsystem (20).

3. Registrierungsverfahren nach einem der vorstehenden Ansprüche, **dadurch gekennzeichnet, dass** bei dem Schritt Erfassen der Lage, bei einem Fehlen oder einer Unterbrechung oder einer Verminderung der Sichtverbindung, die Erfassung der Lage des Roboterkopfs (8) mit der Roboter-Kamera (10) über das Roboterkinematik-Trackingsystem (20) erfolgt, wobei eine Transformation zwischen einer Roboterbasis (4) des Roboters (2) und der Trackingkamera (14) verwendet wird, um über die Transformation von der Trackingkamera (14) zu der Roboterbasis (2) und einer Transformation des Roboterkinematik-Trackingsystems (20) von der Roboterbasis (2) zum Roboterkopf (8) der Roboter-Kamera (10), eine Transformation zwischen dem lokalen Koordinatensystem der Trackingkamera (14) und dem lokalen Koordinatensystem der Roboter-Kamera (10) zu bestimmen und damit deren Lage zu bestimmen.

4. Registrierungsverfahren nach einem der vorstehenden Ansprüche, **dadurch gekennzeichnet, dass** das Registrierungsverfahren ferner den Schritt aufweist:
Erfassen einer relativen Lage des Roboterkopfs (8) mit der Roboter-Kamera (10), insbesondere über einen Roboter-Kamera-Tracker (18), zu der Lage des Kopfs des Patienten (P), insbesondere über einen Patienten-Tracker (16), über die Trackingkamera (14), um dem Roboter (2) eine Orientierung für die automatische Registrierung bereitzustellen.

5. Registrierungsverfahren nach einem der vorstehenden Ansprüche, **dadurch gekennzeichnet, dass** das Registrierungsverfahren ferner den Schritt aufweist:
Verwenden einer 3D-Kamera mit Tiefeninformationen als Roboter-Kamera (10) und Scannen eines Gesichts des Patienten, insbesondere eines Kopfs des Patienten, von mehreren Seiten, um eine 3D-Oberflächen-Gesamtaufnahme des Gesichts, insbesondere des Kopfs zu erhalten.

6. Registrierungsverfahren nach einem der vorstehenden Ansprüche, **dadurch gekennzeichnet, dass** der Schritt der Registrierung des Patienten (P) durchgeführt wird, wenn die Aufnahmen erstellt wurden und die Roboter-Kamera (10) sich wieder im Sichtbereich der Trackingkamera (14) befindet.

7. Chirurgisches Navigationssystem (1) für einen chirurgischen Eingriff bei einem Patienten (P), aufweisend:
einen Roboter (2) mit einer Roboterbasis (4), einem an die Roboterbasis (4) angebundenen Roboterarm (6) sowie einem an den Roboterarm (6) angebundenen, insbesondere gelagerten, Roboterkopf (8) mit einer Roboter-Kamera (10), so dass eine Lage der Roboter-Kamera (10) gegenüber der Roboterbasis (4) einstellbar ist;
ein Trackingsystem (12) mit einer Trackingkamera (14), die vorzugsweise für eine Nachverfolgung einer Position und/oder Orientierung eines Patienten (P), insbesondere eines Kopfs des Patienten (P), vorzugsweise über einen Patienten-Tracker (16), und für eine Nachverfolgung einer Lage der Roboter-Kamera (10) angepasst ist, insbesondere über einen Roboter-Kamera-Tracker (18);
ein Roboterkinematik-Trackingsystem (20), das dafür angepasst ist, eine relative Lage des Roboterkopfs (8) mit der Roboter-Kamera (10) gegenüber seiner Roboterbasis (4) zu bestimmen; und
einer Steuereinheit (22), die dafür angepasst ist, den Roboter (2) zu steuern und eine Registrierung des Patienten (P) durchzuführen, wobei die Steuereinheit (22) konfiguriert ist:
in einer ersten Lage der Roboter-Kamera (10) eine Aufnahme durch die Roboter-Kamera (10) zu erstellen, insbesondere die Roboter-Kamera (10) auf ein Gesicht des Patienten (P) ausrichten und eine Aufnahme von dem Gesicht des Patienten (P) zu erstellen;
bei Vorliegen einer Sichtverbindung zwischen der Trackingkamera (14) und dem Roboterkopf (8) mit der Roboter-Kamera (10), insbesondere mit dem Roboter-Kamera-Tracker (18), die Lage der Roboter-Kamera (10) durch die Trackingkamera (14) zu erfassen oder, bei Fehlen oder Unterbrechung oder Verminderung der Sichtverbindung, die Lage der Roboter-Kamera (10) über das Roboterkinematik-Trackingsystem (20) zu erfassen; und
auf Basis der Aufnahme und der erfassten Lage eine Registrierung des Patienten (P) und/oder der Aufnahme gegenüber dem Navigationssystem (1), insbesondere der Trackingkamera (14), durchzuführen.

8. Chirurgisches Navigationssystem (1) nach Anspruch 7, **dadurch gekennzeichnet, dass** das chirurgische Navigationssystem (1) ein robotergeführtes Operationsmikroskop (24) als Roboter (2) aufweist und ein Mikroskopkopf (26) des Operationsmikroskops (24) als Roboterkopf (8) mit Roboter-Kamera (10) fungiert.

9. Chirurgisches Navigationssystem (1) nach einem der Ansprüche 7 oder 8, **dadurch gekennzeichnet, dass** das chirurgische Navigationssystem (1) ein infrarotbasiertes optisches Trackingsystem (12) aufweist und/oder ein EM-basiertes Trackingsystem aufweist und/oder ein auf Machine-Vision-basiertes Trackingsystem aufweist.

10. Computerlesbares Speichermedium, das Befehle umfasst, die bei einer Ausführung durch einen Computer diesen veranlassen, die Verfahrensschritte des Registrierungsverfahrens gemäß den Ansprüchen 1 bis 6 auszuführen.

## Claims

1. A registration method for an automatic registration of a patient (P) for a surgical navigation, comprising the steps:
preferably detecting a position and/or orientation (S1) of the patient (P), in particular of a head of the patient (P), by a tracking camera (14), in particular a 3D tracking camera, of a surgical navigation system (1);
detecting a pose (S2) of a robot head (8) with a robot camera (10) of a medical robot (2), in particular via a robot camera tracker (18), by the tracking camera (14) of the navigation system (1) and following the robot camera (10);
controlling and moving (S3) a robot arm (6), by a control unit (22), such that the robot camera (10) in a first pose is directed to a region of interest of the patient (P), in particular to a face of the patient (P), and creating (S4) an image, in particular a 3D image, by the robot camera (10);
checking (B5), by the control unit (22), whether there is a visual connection between the tracking camera (14) and the robot head (8) with the robot camera (10), in particular with the robot-camera tracker (18), in the first pose;
detecting (S6.1), when there is a visual connection, a pose of the robot head (8) with the robot camera (10) via the tracking camera (14), or detecting (S6.2), when the visual connection is missing or interrupted or reduced, a pose of the robot head (8) with the robot camera (10) via a robot-kinematics tracking system (20); and
executing (S7) a registration of the patient and/or of the image with respect to the navigation system (1), in particular with respect to the tracking camera (14).

2. The registration method according to claim 1, **characterized in that** the registration method further comprises the steps of:
controlling and moving the robot arm (6) such that the robot camera (10) in a second pose is directed to a further region of interest of the patient (P), in particular to a further facial region of the patient (P), and creating a second image by the robot camera (10);
checking whether in the second pose there is a visual connection between the tracking camera (14) and the robot head (8) with the robot camera (10), in particular with the robot-camera tracker (18);
detecting, when there is a visual connection, the second pose of the robot head (8) with the robot camera (10) via the tracking camera (14), or detecting, when the visual connection is missing or interrupted or reduced, a pose of the robot head (8) with the robot camera (10) via the robot-kinematics tracking system (20).

3. The registration method according to one of the preceding claims, **characterized in that,** in the step of detecting the pose, when a visual connection is missing or interrupted or reduced, the detection of the pose of the robot head (8) with the robot camera (10) is performed via the robot-kinematics tracking system (20), wherein a transformation between a robot base (4) of the robot (2) and the tracking camera (14) is used in order to determine a transformation between the local coordinate system of the tracking camera (14) and the local coordinate system of the robot camera (10) and thus its pose via the transformation from the tracking camera (14) to the robot base (2) and a transformation of the robot-kinematics tracking system (20) from the robot base (2) to the robot head (8) of the robot camera (10).

4. The registration method according to one of the preceding claims, **characterized in that** the registration method further comprises the step of:
detecting a relative pose of the robot head (8) with the robot camera (10), in particular via a robot-camera tracker (18), to the pose of the head of the patient (P), in particular via a patient tracker (16), via the tracking camera (14), in order to provide the robot (2) with an orientation for automatic registration.

5. The registration method according to one of the preceding claims, **characterized in that** the registration method further comprises the step of:
using a 3D camera with depth information as a robot camera (10) and scanning a face of the patient, in particular a head of the patient, from multiple sides in order to obtain an overall 3D surface image of the face, in particular of the head.

6. The registration method according to one of the preceding claims, **characterized in that** the step of registering the patient (P) is performed when the images have been created and the robot camera (10) is again in the field of view of the tracking camera (14).

7. A surgical navigation system (1) for a surgical intervention on a patient (P), comprising:
a robot (2) with a robot base (4), a robot arm (6) connected to the robot base (4) and a robot head (8) connected to the robot arm (6), in particular mounted, with a robot camera (10), so that a pose of the robot camera (10) relative to the robot base (4) is adjustable;
a tracking system (12) with a tracking camera (14) preferably adapted for following a position and/or orientation of a patient (P), in particular a head of the patient (P), preferably via a patient tracker (16), and for following a pose of the robot camera (10), in particular via a robot-camera tracker (18);
a robot-kinematics tracking system (20) adapted to determine a relative pose of the robot head (8) with the robot camera (10) with respect to its robot base (4); and
a control unit (22) adapted to control the robot (2) and to perform registration of the patient (P), wherein the control unit (22) is configured:
in a first pose of the robot camera (10), to create an image by the robot camera (10), in particular to align the robot camera (10) with a face of the patient (P) and to create an image of the face of the patient (P);
when there is a visual connection between the tracking camera (14) and the robot head (8) with the robot camera (10), in particular with the robot-camera tracker (18), to detect the pose of the robot camera (10) by the tracking camera (14) or, when the visual connection is missing or interrupted or reduced, to detect the pose of the robot camera (10) via the robot-kinematics tracking system (20); and
to perform a registration of the patient (P) and/or of the image with respect to the navigation system (1), in particular the tracking camera (14), on the basis of the image and the detected pose.

8. The surgical navigation system (1) according to claim 7, **characterized in** robot (2) and a microscope head (26) of the surgical microscope (24) functions as robot head (8) with robot camera (10).

9. The surgical navigation system (1) according to one of claims 7 or 8, **characterized in that** the surgical navigation system (1) comprises an infrared-based optical tracking system (12) and/or comprises an EM-based tracking system and/or comprises a machine vision-based tracking system.

10. A computer-readable storage medium comprising instructions which, when executed by a computer, cause the computer to perform the method steps of the registration method according to claims 1 to 6.

## Revendications

1. Procédé d'enregistrement pour un enregistrement automatique d'un patient (P) pour une navigation chirurgicale, présentant les étapes suivantes :
de préférence, détection d'une position et/ou d'une orientation (S1) du patient (P), en particulier d'une tête du patient (P), par une caméra de suivi (14), en particulier une caméra de suivi 3D, d'un système de navigation chirurgicale (1) ;
détection d'une position (S2) d'une tête de robot (8) avec une caméra de robot (10) d'un robot médical (2), en particulier via un dispositif de suivi de caméra de robot (18), par la caméra de suivi (14) du système de navigation (1), et suivi de la caméra de robot (10) ;
commande et déplacement (S3), par un dispositif de commande (22), d'un bras de robot (6), de sorte que la caméra de robot (10) est orientée, dans une première position, sur une zone d'intérêt du patient (P), en particulier sur un visage du patient (P), et acquisition (S4) d'une image, en particulier d'une image 3D, par la caméra de robot (10) ;
vérification (B5), par le dispositif de commande (22), de l'existence, dans la première position, d'une liaison visuelle entre la caméra de suivi (14) et la tête de robot (8) avec la caméra de robot (10), en particulier avec le dispositif de suivi de caméra de robot (18) ;
détection (S6.1), en cas de présence d'une liaison visuelle, d'une position de la tête de robot (8) avec la caméra de robot (10) via la caméra de suivi (14), ou détection (S6.2), en cas d'absence ou d'interruption ou de diminution d'une liaison visuelle, d'une position de la tête de robot (8) avec la caméra de robot (10) via un système de suivi cinématique de robot (20) ; et
réalisation (S7) d'un enregistrement du patient et/ou de l'image par rapport au système de navigation (1), en particulier par rapport à la caméra de suivi (14).

2. Procédé d'enregistrement de la revendication 1, **caractérisé en ce que** le procédé d'enregistrement présente de plus les étapes suivantes :
commande et déplacement du bras de robot (6), de sorte que la caméra de robot (10) est orientée, dans une seconde position, sur une autre zone d'intérêt du patient (P), en particulier sur une autre zone du visage du patient (P), et acquisition d'une seconde image par la caméra de robot (10) ;
vérification de l'existence, dans la seconde position, d'une liaison visuelle entre la caméra de suivi (14) et la tête de robot (8) avec la caméra de robot (10), en particulier du dispositif de suivi de caméra de robot (18) ;
détection, en cas de présence d'une liaison visuelle, d'une seconde position de la tête de robot (8) avec la caméra de robot (10) par la caméra de suivi (14), ou détection, en cas d'absence ou d'interruption ou de diminution d'une liaison visuelle, d'une position de la tête de robot (8) avec la caméra de robot (10) par un système de suivi cinématique de robot (20).

3. Procédé d'enregistrement selon l'une des revendications précédentes, **caractérisé en ce que,** lors de l'étape de détection de la position, en cas d'absence ou d'une interruption ou d'une diminution de la liaison visuelle, la détection de la position de la tête de robot (8) avec la caméra de robot (10) est effectuée via le système de suivi cinématique de robot (20), dans lequel une transformation entre une base de robot (4) du robot (2) et la caméra de suivi (14) est utilisée afin de déterminer, via la transformation de la caméra de suivi (14) à la base de robot (2) et via une transformation du système de suivi cinématique de robot (20) de la base de robot (2) à la tête de robot (8) de la caméra de robot (10), une transformation entre le système de coordonnées local de la caméra de suivi (14) et le système de coordonnées local de la caméra de robot (10) et de déterminer ainsi leur position.

4. Procédé d'enregistrement selon l'une des revendications précédentes, **caractérisé en ce que** le procédé d'enregistrement présente de plus l'étape suivante :
détection d'une position relative de la tête de robot (8) avec la caméra de robot (10), en particulier via un dispositif de suivi de caméra de robot (18), par rapport à la position de la tête du patient (P), en particulier via un dispositif de suivi de patient (16), via la caméra de suivi (14), afin de fournir au robot (2) une orientation pour l'enregistrement automatique.

5. Procédé d'enregistrement selon l'une des revendications précédentes, **caractérisé en ce que** le procédé d'enregistrement présente de plus l'étape suivante :
utilisation d'une caméra 3D à informations de profondeur en tant que caméra de robot (10) et balayage d'un visage de patient, en particulier d'une tête de patient, sous plusieurs angles, afin d'obtenir une image 3D globale de la surface du visage, en particulier de la tête.

6. Procédé d'enregistrement selon l'une des revendications précédentes, **caractérisé en ce que** l'étape d'enregistrement du patient (P) est réalisée une fois que les acquisitions d'images ont été réalisées et que la caméra de robot (10) se trouve à nouveau dans le champ de vision de la caméra de suivi (14).

7. Système de navigation chirurgical (1) pour une intervention chirurgicale auprès d'un patient (P), comprenant :
un robot (2) avec une base de robot (4), un bras de robot (6) relié à la base de robot (4), ainsi qu'une tête de robot (8), reliée au bras de robot (6), en particulier montée sur celui-ci, avec une caméra de robot (10), de sorte qu'une position de la caméra de robot (10) est réglable par rapport à la base de robot (4) ;
un système de suivi (12) avec une caméra de suivi (14) qui est adapté de préférence pour un suivi d'une position et/ou d'une orientation d'un patient (P), en particulier d'une tête de patient (P), de préférence au moyen d'un dispositif de suivi de patient (16), et pour un suivi d'une position de la caméra de robot (10), en particulier au moyen d'un dispositif de suivi de caméra de robot (18) ;
un système de suivi cinématique de robot (20) qui est adapté pour déterminer une position relative de la tête de robot (8) avec la caméra de robot (10) par rapport à sa base de robot (4) ; et
un dispositif de commande (22) adapté pour commander le robot (2) et réaliser un enregistrement du patient (P), le dispositif de commande (22) étant configuré pour :
dans une première position de la caméra de robot (10), acquérir une image au moyen de la caméra de robot (10), en particulier orienter la caméra de robot (10) sur un visage de patient (P) et acquérir une image du visage du patient (P) ;
en cas de présence d'une liaison visuelle entre la caméra de suivi (14) et la tête de robot (8), détecter, avec la caméra de robot (10), en particulier avec le dispositif de suivi de caméra de robot (18), la position de la caméra de robot (10) par la caméra de suivi (14) ou en cas d'absence ou d'interruption ou de diminution de la liaison visuelle, détecter la position de la caméra de robot (10) via le système de suivi cinématique de robot (20) ; et
réaliser, sur la base de l'acquisition et de la position détectée, un enregistrement du patient (P) et/ou de l'image par rapport au système de navigation (1), en particulier par rapport à la caméra de suivi (14).

8. Système de navigation chirurgical (1) selon la revendication 7, **caractérisé en ce que** le système de navigation chirurgical comprend un microscope opératoire robotisé (24) en tant que robot (2), et **en ce qu'**une tête de microscope (26) du microscope opératoire (24) fait fonction de tête de robot (8) avec caméra de robot (10).

9. Système de navigation chirurgical (1) selon l'une des revendications 7 ou 8, **caractérisé en ce que** le système de navigation chirurgical (1) présente un système de suivi optique basé sur infrarouge (12) et/ou un système de suivi basé sur l'EM et/ou un système de suivi basé sur la vision par ordinateur.

10. Support de stockage lisible par ordinateur, qui comprend des instructions qui, lorsqu'elles sont exécutées par un ordinateur, amènent celui-ci à exécuter les étapes de procédé du procédé d'enregistrement selon les revendications 1 à 6.
